# EUROPÄISCHE PATENTSCHRIFT

(11) **EP 3 266 757 B1**
(45) Veröffentlichungstag und Bekanntmachung des Hinweises auf die Patenterteilung: **25.12.2019**
(21) Anmeldenummer: 16178531.6
(22) Anmeldetag: 08.07.2016
(51) Int. Cl.: C07C 1/20, C07C 2/10, C07C 2/24, C07C 2/26, C07C 41/06, C07C 11/02, C07C 11/08, C07C 11/107, C07C 43/04

(54) **HERSTELLUNG VON ZUMINDEST 1-HEXEN UND OCTEN AUS ETHEN**
PRODUCING AT LEAST 1-HEXENE AND OCTEN FROM ETHENE
FABRICATION COMBINEE D'AU MOINS 1 HEXENE ET D'OCTENE A PARTIR D'ETHYLENE

(43) Veröffentlichungstag der Anmeldung: 10.01.2018
(73) Patentinhaber: Evonik Operations GmbH, 45128 Essen (DE)
(72) Erfinder: STOCHNIOL, Guido, 45721 Haltern am See (DE); REEKER, Helene, 44227 Dortmund (DE); MASCHMEYER, Dietrich, 45657 Recklinghausen (DE); PEITZ, Stephan, 45739 Oer-Erkenschwick (DE); SCHALLENBERG, Jörg, 46286 Dorsten (DE)
(74) Vertreter: Evonik Patent Association

(56) Entgegenhaltungen:
- WO-A1-95/11874
- WO-A1-2015/083053

## Beschreibung

Die Erfindung befasst sich mit der Herstellung von 1-Hexen und Octenen aus Ethen. Optional soll auch 1-Buten hergestellt werden.

Kohlenwasserstoffe sind chemische Verbindungen, die ausschließlich aus Kohlenstoff und Wasserstoff bestehen. Alkene (synonym: Olefine) sind Kohlenwasserstoffe, die eine C=C Doppelbindung im Molekül aufweisen. Alkane (synonym: Paraffine) sind dagegen Kohlenwasserstoffe, die nur Einfachbindungen aufweisen. Sie werden deswegen auch als gesättigt bezeichnet.

In der organischen Chemie werden Kohlenwasserstoffe häufig nach der Anzahl ihrer Kohlenstoffatome pro Molekül bezeichnet, indem der jeweiligen Substanzklasse das Präfix Cₙ vorangestellt wird. Dabei bezeichnet n die jeweilige Anzahl der Kohlenstoffatome in einem Molekül. So sind C₄-Olefine zu verstehen als Substanzen der Klasse der Alkene mit vier Kohlenstoffatomen. C₈-Olefine weisen dementsprechend acht Kohlenstoffatome pro Molekül auf. Soweit im Folgenden das Präfix Cₙ₊ verwendet wird, ist von einer Substanzklasse die Rede, die mehr als n Kohlenstoffatome pro Molekül aufweist. Ein C₄₊-Olefin hat demnach mindestens fünf Kohlenstoffatome.

Das einfachste Olefin ist Ethen (Ethylen). Es weist zwei Kohlenstoffatome auf. Ethen stellt eine bedeutsame Grundchemikalie dar und wird daher in großen Mengen hergestellt. Dies geschieht meist durch Spaltung von Naphtha. Darüber hinaus kann es durch Dehydrierung von Ethan gewonnen werden, welches wiederum ein Bestandteil des Erdgases ist. Aufgrund zunehmender Erschließung von unkonventionellen Erdgasquellen und abnehmender Erdöl-Förderung nimmt der Anteil an auf Erdgas basierendem Ethen stetig zu. Die physikalischen Eigenschaften von Ethen und dessen Herstellung sind beschrieben in:
Zimmermann, Heinz und Walzl, Roland: Ethylene. Ullmann's Encyclopedia of Industrial Chemistry (2009).

Unter der Oligomerisierung versteht man die Reaktion von Kohlenwasserstoffen mit sich selbst, wobei entsprechend längerkettige Kohlenwasserstoffe, die so genannten Oligomere, entstehen. Olefine mit zwei bis acht Kohlenstoffatomen lassen sich recht gut oligomerisieren.

So lässt sich beispielsweise durch die Oligomerisierung von zwei Olefinen mit drei Kohlenstoffatomen Olefine mit sechs Kohlenstoffatomen (Hexen) aufbauen. Die Oligomerisierung von zwei Molekülen miteinander wird auch Dimerisierung genannt. Verbinden sich hingegen drei Olefine mit drei Kohlenstoffatomen miteinander (Trimerisierung), entsteht ein Olefin mit neun Kohlenstoffatomen. Die Tetramerisierung von Ethen führt zu Octenen, das sind Olefine mit acht Kohlenstoffatomen.

Beachtlich ist, dass bei der Oligomerisierung stets ein Gemisch aus unterschiedlichen Oligomeren (Oligomerisat) entsteht. So entstehen bei der Oligomerisierung von Ethen nicht nur Ethen-Dimere, sondern parallel dazu auch Trimere und Tetramere. Das Oligomerisat enthält also eine Bandbreite von unterschiedlich langen C₂₊-Olefinen.

Darüber hinaus liegen die Oligomere einer Kohlenstoffatomanzahl in unterschiedlichen isomeren Strukturen vor: So werden bei der Dimerisierung von Ethen die drei isomeren C₄-Olefine 1-Buten, cis-2-Buten und trans-2-Buten gebildet, währenddessen bei der Trimerisierung von Ethen bis zu zehn unterschiedliche C₆-Olefin-Isomere entstehen können, nämlich 1-Hexen, (E)-2-Hexen, (Z)-2-Hexen, (E)-3-Hexen, (Z)-3-Hexen, (R)-3-Methyl-1-penten, (S)-3-Methyl-1-penten, (*E*)-3-Methyl-2-penten, (Z)-3-Methyl-2-penten und 2-Ethyl-1-buten. Die Vielfalt der durch C₂-Tetramerisierung gebildeten C₈-Olefine ist noch größer.

Es lässt sich also festhalten, dass durch Oligomerisierung von Ethen ein hochkomplexes Gemisch unterschiedlicher Olefine entsteht.

Von industriellem Interesse sind dabei jedoch nur die Substanzen 1-Buten und 1-Hexen, die als Monomer oder Co-Monomer bei der Herstellung von Kunststoffen eingesetzt werden. Olefinen mit acht Kohlenstoffatomen lassen sich durch Hydroformylierung und Hydrierung in C₉-Alkohole überführen, die wiederum als Ausgangsstoff für die Produktion von diversen PVC-Weichmachern dienen.

Wer Ethen oligomerisiert, wird somit mit dem Problem konfrontiert, die wenigen begehrten Substanzen aus dem Oligomerisat abzutrennen.

Solange Olefine mit unterschiedlicher Anzahl Kohlenstoffatome zu trennen sind, ist dies mit thermischen Trennverfahren (Destillation) problemlos möglich, da aus den unterschiedlichen Kettenlängen weit auseinander liegende Siedepunkte resultieren. Ein solch hartes Trennkriterium liegt bei Gemischen von Isomeren mit identischer Kohlenstoffatomanzahl hingegen nicht vor, weil die Siedepunkte der Isomere meist sehr dicht beieinander liegen. Eine destillative Trennung ist dann nur mit großem apparativen Aufwand und hohem Energieeinsatz möglich, sodass die Trennung im Ergebnis viel zu teuer ist.

So liegt beispielsweise die Siedetemperatur von 1-Hexen bei 63°C und von Isohexen (korrekte Bezeichnung: 4-Methyl-1-penten) bei 54°C. Dieser geringe Unterschied in der Siedetemperatur macht es unwirtschaftlich, die beiden Reinsubstanzen 1-Hexen und Isohexen aus einem Gemisch destillativ zu gewinnen; dies gilt umso mehr, wenn es sich nicht um ein binäres Gemisch dieser beiden Substanzen handelt sondern um ein Oligomerisat mit einer Vielzahl weiterer C₆-Olefin-Isomere, deren jeweilige Siedepunkte sich ebenfalls in diesem engen Temperaturfenster bewegen.

Um eine Isohexen und 1-Hexen wirtschaftlich trennen wendet US2010/0099934A1 einen Trick an: Das C₆-Olefin-Gemisch wird einem Veretherungsschritt unterworfen, bei dem das Isohexen mit Methanol in Ether umgesetzt wird.

In der Veretherung wird deutlich mehr Isohexen umgesetzt als 1-Hexen. Dadurch wird ein Reaktionsgemisch erhalten, welches neben 1-Hexen den Ether 3-Methyl-3-methoxypentan anstelle des Isohexens enthält. Da 3-Methyl-3-methoxypentan - genannt auch Methyl-tert. hexyl ether (MTHxE) - höher siedet als das 1-Hexen, lässt sich das 1-Hexen mit wenig Aufwand destillativ von dem MTHxE abtrennen. Auf diese Weise kann über die selektive Isohexen-Veretherung reines 1-Hexen aus C₆-Olefingemischen gewonnen werden, wie sie bei der Ethen-Oligomerisierung anfallen.

In Hinblick auf den in US2010/0099934A1 beschriebenen Prozess stellt sich die Frage, was mit dem abgetrennten MTHxE geschieht. Es liegt nahe, dieses Ethergemisch als Anti-Klopfmittel in den Kraftstoffpool einzuspeisen, so etwa wie es US5752992 lehrt. Die darin gebundenen Kohlenstoffatome werden somit letztendlich verbrannt und stehen als Ausgangsstoff für hochwertige Spezialchemikalien nicht mehr zur Verfügung.

Darüber hinaus ist anzumerken, dass die Ethen-Oligomerisierung gar kein Isohexen (4-Methyl-1-penten) hervor bringt, sondern lediglich die zehn oben aufgezählten C₆-Olefine. Das in US2010/0099934A1 gelöste technische Problem einer Trennung von 1-Hexen und Isohexen tritt folglich bei der Aufarbeitung von Ethen-Oligomerisaten gar nicht auf; es ist vielmehr dann zu lösen, wenn es gilt C₆-Olefinmischungen zu verwerten, die aus der Metathese von C₄-Olefinen stammen.

Weiterhin offenbart die WO 2015/083053 A1 ein Verfahren zu Oligomerisierung on Ethen unter Verwendung eines chromhaltigen Oligomerisierungskatalysators. Das Verfahren umfasst insbesondere die Verwendung von zwei unterschiedlichen Liganden, die an einem Stickstoffatom unterschiedliche Substituenten aufweisen.

In der WO 95/11874 A1 wird außerdem ein Verfahren zur Herstellung von 3-Methyl-2-penten, wobei das Reaktionsprodukt im Wesentlichen keine weiteren C6-Olefine außer 2-Ethyl-1-buten umfasst. Das dort beschriebene Verfahren umfasst im ersten Schritt eine Trimerisierung von Ethen, im zweiten Schritt eine Veretherung mit anschließender Spaltung des Ethers, um 3-Methyl-2-penten zu erhalten.

Im Lichte dieses Standes der Technik macht es sich die vorliegende Erfindung zur Aufgabe, das bekannte Verfahren zur Herstellung von 1-Hexen aus Ethen dahingehend weiterzubilden, dass die die eingesetzten Kohlenstoffatome chemisch besser ausgenutzt werden.

Gelöst wird diese Aufgabe dadurch, dass MTHxE katalytisch zurückgespalten wird in die C₆-Olefine und den Alkohol, dass der Alkohol bei der Veretherung wiederverwertet wird und dass die erhaltenen C₆-Olefine mit Ethen in C₈-Olefine umgesetzt werden. Auf diese Weise geht der der Alkohol aus dem Prozess nicht verloren sondern wird als Derivatisierungsmittel intern im Kreis geführt. Die weniger attraktiven C₆-Olefine aus dem Spaltprodukt werden mit weiterem Ethen zu Octen aufgewertet, sodass ein weiteres Verkaufsprodukt zur Verfügung steht.

Konkret ist ein Verfahren zur Herstellung von zumindest 1-Hexen und Octen aus Ethen mit den folgenden Schritten Gegenstand der Erfindung:
a) Bereitstellen eines Einsatzstoffgemisches enthaltend Ethen;
b) Unterwerfen des Ethens einer ersten Oligomerisierung, wobei ein erstes Oligomerisat erhalten wird, welches Olefine mit weniger als sechs Kohlenstoffatome, zumindest die C₆-Olefine 1-Hexen, , 3-Methyl-cis-2-penten, 3-Methyl-trans-2-penten und Olefine mit mehr als sechs Kohlenstoffatome enthält,
c) Destillatives Aufarbeiten des ersten Oligomerisats, wobei eine C₆-Fraktion gewonnen wird, die zumindest 1-Hexen, 3-Methyl-cis-2-penten, 3-Methyl-trans-2-penten enthält,
d) Unterwerfen der C₆-Fraktion einer Veretherung unter Zugabe eines Alkohols in Gegenwart eines heterogenen Katalysators unter Erhalt eines ersten Reaktionsgemisches, enthaltend zumindest 1-Hexen und 3-Methyl-3-methoxypentan;
e) Destillatives Aufarbeiten des ersten Reaktionsgemisches unter Erhalt von einer ersten Zielfraktion enthaltend 1-Hexen sowie einer Etherfraktion mindestens enthaltend 3-Methyl-3-methoxypentan;
f) wobei die Etherfraktion einer Spaltreaktion unterworfen wird, wodurch ein Spaltprodukt erhalten wird, welches 3-Methyl-cis-2-penten, 3-Methyl-trans-2-penten sowie den Alkohol enthält;
g) wobei der Alkohol aus dem Spaltprodukt destillativ abgetrennt und in der Veretherung wiederverwertet wird;
h) wobei ein erstes Intermediat mindestens enthaltend 3-Methyl-cis-2-penten, 3-Methyl-trans-2-penten aus dem Spaltprodukt destillativ abgetrennt wird;
i) wobei das erste Intermediat mit Ethen umgesetzt wird, sodass ein zweites Reaktionsgemisch erhalten wird, welches Olefine mit zumindest acht Kohlenstoffatome enthält;
k) und wobei eine zweite Zielfraktion enthaltend Octen aus dem zweiten Reaktionsgemisch destillativ gewonnen wird,
wobei ein mindestens zwei Komponenten enthaltender Festkörper in der ersten Oligomerisierung und/oder in der zweiten Oligomerisierung als heterogener Katalysator eingesetzt wird, wobei die erste Komponente mindestens ein aus Ni, Fe, Ti ausgewähltes Element umfasst, welches in metallischer und/oder oxidischer und/oder hydridischer Form vorliegt, und wobei die zweite Komponente mindestens ein aus Al₂O₃, SiO₂, TiO₂, ZrO₂ ausgewähltes Metalloxid umfasst.

Die Erfindung beruht auf der Erkenntnis, dass die Veretherung reversibel ist und dass sich aus Reaktion von C₆-Olefine mit Ethen Octene herstellen lassen. Durch geschickte Integration dieser Verfahrensschritte in den Destillationsaufbau lassen sich die Wertprodukte mit wenig Zusatzaufwand gewinnen; dazu später mehr.

In einer besonders bevorzugten Weiterbildung des hier beschriebenen Verfahrens wird aus dem Ethen neben 1-Hexen und Octen auch 1-Buten als drittes Zielprodukt hergestellt. Dies geschieht folgendermaßen:
a) die erste Oligomerisierung wird dergestalt durchgeführt, dass das erste Oligomerisat 1-Buten, cis-2-Buten und trans-2-Buten enthält;
b) beim destillativen Aufarbeiten des ersten Oligomerisats wird eine C₄-Fraktion gewonnen, die 1-Buten, cis-2-Buten und trans-2-Buten enthält;
c) die C₄-Fraktion wird destillativ getrennt in eine dritte Zielfraktion enthaltend 1-Buten und in ein zweites Intermediat, welches cis-2-Buten und trans-2-Buten enthält;
d) das zweite Intermediat wird einer zweiten Oligomerisierung unterworfen, wobei ein zweites Oligomerisat erhalten wird, welches Olefine mit zumindest acht Kohlenstoffatome enthält;
e) das zweite Oligomerisat wird gemeinsam mit dem zweiten Reaktionsgemisch destillativ aufgearbeitet, wobei die zweite Zielfraktion gewonnen wird.

Sofern bei der Oligomerisierung von Ethen die in Schritt a) genannten Butene anfallen (ist wird in der Regel der Fall sein), ist es mit dieser Erweiterung möglich, das begehrte 1-Buten als drittes Zielprodukt zu gewinnen. Das weniger brauchbare 2-Buten wird durch die zweite Oligomeriserung in die Cs-Fraktion überführt und der ohnehin vorhandenen Aufarbeitung unterzogen.

Bei der Oligomerisierung des Ethens werden auch Olefine mit mehr als sechs Kohlenstoffatome anfallen. Diese können gemeinsam mit den entsprechenden Olefinen aus dem zweiten Reaktionsgemisch aufgearbeitet werden, um Apparatekosten zu sparen. Eine Weiterbildung des Verfahrens sieht es demnach vor, dass beim destillativen Aufarbeiten des ersten Oligomerisats eine C₆₊-Fraktion gewonnen wird, die Olefine mit mehr als sechs Kohlenstoffatome enthält, und dass die C₆₊-Fraktion gemeinsam dem zweiten Reaktionsgemisch destillativ aufgearbeitet wird, wobei die zweite Zielfraktion gewonnen wird.

Unter der Maßgabe, dass auch 1-Buten aus Ethen hergestellt wird, entstehen bei der Oligomerisierung des 2-Butens ebenfalls Olefine mit der Kettenlänge C₆+, die kostengünstig gemeinsam aufgearbeitet werden können. Eine entsprechende Weiterbildung des Verfahrens sieht demnach vor, dass beim destillativen Aufarbeiten des ersten Oligomerisats eine C₆₊-Fraktion gewonnen wird, die Olefine mit mehr als sechs Kohlenstoffatome enthält, und dass die C₆₊-Fraktion gemeinsam dem zweiten Reaktionsgemisch und mit dem zweiten Oligomerisat destillativ aufgearbeitet wird, wobei die zweite Zielfraktion gewonnen wird.

Die Oligomerisierung des Ethens kann in der Flüssigphase oder in der Gasphase stattfinden. Sofern eine Gasphasen-Oligomerisierung des Ethens durchgeführt wird sollte das Einsatzstoffgemisch mehr als 95 Gew.-% Ethen enthalten, und die Reaktionbedingungen der ersten Oligomerisierung so zu wählen, dass die erste Oligomerisierung in der Gasphase erfolgt. Als Katalysator ist dann ein Festkörper notwendig (heterogene Katalyse).

Besser ist, die erste Oligomerisierung in der Flüssigphase in Gegenwart eines Festkörper-Katalysators durchzuführen, weil dann die Prozessintensität der Reaktion größer ist. Um Ethen flüssig zu oligomerisieren, gibt es verschiedene Möglichkeiten:
Eine erste besteht darin, das Ethen in einem Lösemittel zu lösen. Entsprechend wird ein Einsatzstoffgemisch verwendet, welches weniger als 25 Gew.-% Ethen enthält und welches mehr als 75 Gew.-% des Lösemittels enthält. Die Reaktionbedingungen der ersten Oligomerisierung und die Konzentration des Ethens in dem Lösemittel sind dann so zu wählen, dass die erste Oligomerisierung in der flüssigen Phase erfolgt und das Ethen in dem flüssigen Lösemittel vollständig gelöst ist. Das Verhältnis von Ethen zu Lösemittel wird dann eingestellt auf einen Wert zwischen 0.1 und 0.5; die Temperatur liegt zwischen 20°C und 150 °C und der Druck zwischen 1*10⁵ Pa und 50*10⁵ Pa. Der Katalysator kann dann wahlweise homogen in dem Lösemittel mit gelöst sein oder bevorzugt als heterogener Katalysator als Feststoff vorliegen.

Als Lösemittel eignen sich grundsätzlich solche Substanzen, die sich in der Oligomerisierung inert verhalten, also an der Reaktion nicht wesentlich teilnehmen. Konkret sind C₃- bis C₇-Alkane bzw. C₃ - bis C₇ - Cycloalkane als Lösemittel geeignet (rein oder gemischt). Da sich das Lösemittel inert verhält, findet es sich in dem ersten Oligomerisat wieder. Es wird destillativ aus dem ersten Oligomerisat abgetrennt und zurückgeführt. Auf diese Weise wird das Lösemittel stets im Kreislauf geführt und geht so nicht verloren. Der Ort der Abtrennung des Lösemittels von dem ersten Oligomerisat hängt von dem Siedepunkt des Lösemittels ab. Wird beispielsweise Propan oder Isobutan als Lösemittel eingesetzt, sieden diese vor den Dimeren des Ethens und werden zuvor abgetrennt. Werden stattdessen n-Hexan oder n-Heptan als Lösemittel verwendet, erfolgt die Abtrennung des Lösemittels hinter der Hexen-Abtrennung.

Sofern im Einsatzstoffgemisch als einzige reaktive Komponente Ethen enthalten ist, kann es abhängig von der gewählten Reaktionsführung zu einem nicht vollständigen Umsatz des Ethens kommen. Überschüssiges und deswegen in der ersten Oligomerisierung nicht umgesetztes Ethen wird destillativ aus dem ersten Oligomerisat abgetrennt und in die erste Oligomerisierung zurückgeführt.

Eine besonderes Ausführungsform des Verfahrens sieht vor, dass das Einsatzstoffgemisch neben Ethen auch Olefine mit vier Kohlenstoffatome enthält, wobei die Reaktionsbedingungen der ersten Oligomerisierung und die Zusammensetzung des Einsatzstoffgemisches so gewählt sind, dass die erste Oligomerisierung in der flüssigen Phase erfolgt und das Ethen in den Olefinen mit vier Kohlenstoffatomen vollständig gelöst ist. Dahinter steht der Gedanke, als Lösemittel ein reaktives Material zu verwenden, nämlich Olefine mit vier Kohlenstoffatome, genauer gesagt 1-Buten, cis-2-Buten und trans-2-Buten. Diese Olefine lassen sich nämlich bei vergleichsweise geringen Drücken verflüssigen und eignen sich so als Lösemittel für das Ethen. Da die C₄-Olefine selbst reaktiv sind, werden sie in der ersten Oligomerisierung umgesetzt in Richtung C₈ und C₈₊. Da die Octene ohnehin als Zielfraktion gewonnen und ausgeschleust werden, erübrigt sich damit eine Kreislaufführung des Lösemittels. Freilich muss stets frisches C₄ Alken der ersten Oligomerisierung zugeführt werden. Da Buten-haltige Stoffgemische als Standard-Rohstoffe der Petro-Folgechemie gehandelt werden, ist dies in den meisten Fällen kein Problem.

Sofern jedoch keine C₄-Quelle zur Verfügung steht, kann als reaktives Lösemittel auch C₆-Olefin verwendet werden, welches im Prozess ohnehin durch Ethen-Trimerisierung entsteht. Eine bevorzugte Variante des Verfahrens sieht daher vor, dass das Einsatzstoffgemisch neben Ethen auch Olefine mit sechs Kohlenstoffatome enthält, wobei die Reaktionsbedingungen der ersten Oligomerisierung und die Zusammensetzung des Einsatzstoffgemisches so gewählt sind, dass die erste Oligomerisierung in der flüssigen Phase erfolgt und das Ethen in den Olefinen mit sechs Kohlenstoffatomen vollständig gelöst ist. Die als reaktive Lösemittel verwendeten Olefine mit sechs Kohlenstoffatome werden vorzugsweise unmittelbar vor der Veretherung abgezweigt und in die Ethen-Oligomerisierung rezykliert. Dies setzt voraus, dass der Prozess genügend Hexen bildet; nur ein Teil der C₆-Fraktion kann als Lösemittel verwendet werden kann, da sonst nicht mehr genug C₆ für die Veretherung zur Verfügung steht.

Erlaubt eine geringe C₆-Bildungsrate es nicht, die als Lösemittel benötigte Menge an Hexen zurückzuführen, kann selbstverständlich auch C₄-Olefin als zweites Lösemittel verwendet werden. Diese Verfahrensvariante ist dadurch gekennzeichnet, dass das Einsatzstoffgemisch neben Ethen auch Olefine mit vier Kohlenstoffatome und Olefine mit sechs Kohlenstoffatome enthält, wobei die Reaktionsbedingungen der ersten Oligomerisierung und die Zusammensetzung des Einsatzstoffgemisches so gewählt sind, dass die erste Oligomerisierung in der flüssigen Phase erfolgt und das Ethen in den Olefinen mit vier und sechs Kohlenstoffatomen vollständig gelöst ist.

Schließlich ist es noch möglich, ein Lösemittelgemisch zu verwenden, das teilweise inert, teilweise reaktiv ist. So könnte beispielsweise eine Mischung aus C₆-Olefin und n-Hexan und/oder aus n-Buten und iso-Buten als Lösemittel verwendet werden. Das Vorgesagte gilt dann entsprechend.

Die erste und/oder die zweite Oligomerisierung und vorzugsweise auch die Umsetzung des zweiten Intermediats mit dem Ethen werden in Gegenwart desselben heterogenen Katalysators durchgeführt. Ein Festkörper, der alle drei Reaktionen zu katalysieren vermag enthält mindestens zwei Komponenten wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus Al₂O₃, SiO₂, TiO₂, ZrO₂ ausgewähltes Metalloxid umfasst. Ein Beispiel eines solchen Katalysators ist aus US2581228 bekannt.

Typische Reaktionsbedingungen für die erste und zweite Oligomerisierung an diesem Katalysator in der Flüssigphase bewegen sich im folgenden Bereich:

| | |
|---|---|
| Temperatur: | 20°C bis 150°C |
| Druck: | 1*10⁵ Pa bis 50*10⁵ Pa |
| Raum/Zeit-Belastung: | 3 h⁻¹ bis 50 h⁻¹ |

Die Umsetzung des zweiten Intermediats mit Ethen in Gegenwart des besagten Katalysators erfolgt bei einer Temperatur zwischen 20 °C und 150 °C und bei einem Druck zwischen 1*10⁵ Pa und 50*10⁵ Pa, wobei das Mengenverhältnis des Ethens gegenüber dem zweiten Intermediat so gewählt ist, dass das zweite Intermediat in der flüssigen Phase vorliegt und das Ethen darin vollständig gelöst ist. Dies gelingt bei einem Verhältnis von Ethen zu Intermediat im Bereich von 0.1 bis 0.5.

Die Veretherung erfolgt in der flüssigen Phase bei einem Druck von 1*10⁵ Pa bis 7*10⁵ Pa und einer Temperatur von 50°C bis 130°C Gegenwart eines Polystyrol-Divinylbenzol-Copolymers erfolgt, welches Sulfonsäuregruppen oder Carbonsäuregruppen trägt. Bei dem genannten Stoff handelt es sich um ein saures Ionenaustauscher-Harz wie zum Beispiel Amberlyst® 15 von Rohm & Haas.

Als Alkohol wird einfachstenfalls Methanol verwendet. Die Umsetzung von Hexen mit Methanol zu MTHxE ist detailliert beschrieben in:
Krause, A.O.I.; Hammarström, L.G.; Joutsimo, M.: Etherification of C6-Olefins with Methanol. In Proceedings of the 8th International Congress on Catalysis, Berlin, Germany, July 2-6, 1984; Vol. V, V553-V563.

Anders als die übrigen Reaktionen wird die die Spaltreaktion Vorzugsweise in der Gasphase durchgeführt, bei einem Druck von 1*10⁵ Pa bis 25*10⁵ Pa und einer Temperatur von 130°C bis 350°C. Die Ether-Spaltung lässt sich heterogen katalysieren mit einem Festkörper, welcher die folgende, sich zu 100 Gew.-% ergänzende Zusammensetzung aufweist:

| | |
|---|---|
| • Siliciumdioxid: | 61 Gew.-% bis 74 Gew.-%; |
| • Aluminiumdioxid: | 19 Gew.-% bis 23 Gew.-%; |
| • Magnesiumoxid: | 10 Gew.-% bis 12 Gew.-%; |
| • Summe sonstige Stoffe: | 0 bis 10 Gew.-%. |

Die Herstellung eines solchen Spalt-Katalysators ist in EP1894621B1 beschrieben.

In der ersten Oligomerisierung wird neben 1-Hexen in den meisten Fällen auch 2-Hexen und 3-Hexen gebildet. Sofern es nicht weiterreagiert, findet es sich dann im ersten Reaktionsgemisch wieder. Die Ausbeute an 1-Hexen des Gesamtprozesses lässt sich dadurch steigern, dass das im ersten Reaktionsgemisch enthaltende 2-Hexen und 3-Hexen von dem 1-Hexen destillativ getrennt und sodann einer Isomerisierung unterzogen wird, welche das 2-Hexen und das 3-Hexen in Richtung des 1-Hexens isomerisiert. Das dabei erhaltende Isomerisat wird wieder in die Abtrennung des 1-Hexens von dem 2-Hexen und 3-Hexen zurückgeführt. Durch Separierung von 2-Hexen und 3-Hexen und dessen Isomerisierung wird die Ausbeute an 1-Hexen gesteigert.

Die Isomerisierung erfolgt in der Gasphase bei einer Temperatur zwischen 100°C und 300°C sowie bei einem Druck zwischen 1*10⁵ Pa und 10*10⁵ Pa. Die Isomerisierung wird heterogen katalysiert an einem festen Katalysator, welcher als katalytisch aktives Material mindestens eines der folgenden Oxide enthält: MgO, CaO, BaO, Li₂O, SrO.

Konkret können in der Isomerisierung auch basische Katalysatoren verwendet werden. Solche basische Katalysatoren enthalten als Hauptkomponenten Aluminiumoxid und/oder Zirkoniumdioxid sowie oben genannte Alkalimetalloxide und/oder Erdalkalimetalloxide. Als weitere Komponenten können im Katalysator Titandioxid, Siliciumdioxid und/oder Thoriumoxid mit 0.01 Gew.-% bis 3 Gew.-%, bevorzugt 0.5 Gew.-% bis 5 Gew.-% enthalten sein. Derartige Materialien sind in WO2004/052809A1 offenbart.

Verschiedene Verfahrensvarianten sollen nun anhand von vereinfachten Fließbildern erläutert werden. Diese zeigen:
- Figur 1:: Basisausführung, Einsatzstoff lediglich Ethen;
- Figur 2:: Ausführung mit Einsatzstoff Ethen und Buten
- Figur 3:: wie Figur 2, zusätzlich mit Rückführung von Überschuss-Methanol
- Figur 4:: Ausführung mit Einsatzstoff lediglich Ethen unter Verwendung von Hexen als Lösemittel;
- Figur 5:: Ausführung mit Einsatzstoff lediglich Ethen unter Verwendung von schwer siedendem Lösemittel;
- Figur 6:: Ausführung mit Einsatzstoff lediglich Ethen unter Verwendung von leicht siedendem Lösemittel.

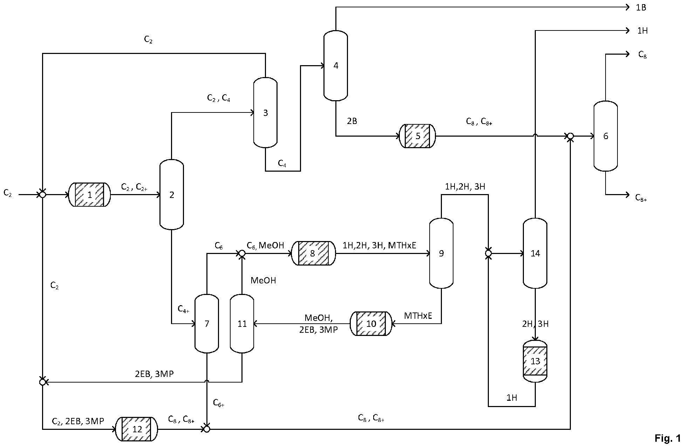

Figur 1 zeigt die einfachste Ausführungsform des Verfahrens. Ethen C₂ wird als Einsatzstoffgemisch bereitgestellt. Reinheit > 95 %. In einer ersten Oligomerisierung 1 wird das Ethen an einem heterogenen Katalysator in der Gasphase oligomerisiert.

Die erste Oligomerisierung 1 erfolgt in Gegenwart eines Festkörpers, der mindestens zwei Komponenten enthält wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus Al₂O₃, SiO₂, TiO₂, ZrO₂ ausgewähltes Metalloxid umfasst.

In der ersten Oligomerisierung 1 entsteht ein erstes Oligomerisat C₂, C₂+ enthaltend die Oligomere C₂+ sowie nicht umgesetztes Ethen C₂. In einer ersten Destillationskolonne werden über Kopf eine Fraktion C₂, C₄ enthaltend das nicht umgesetzte Ethen sowie die Ethen-Dimere abgetrennt, währenddessen die Olefine mit mehr als vier Kohlenstoffatome C₄+ aus dem Sumpf der ersten Destillationskolonne 2 abgezogen werden.

In einer zweiten Destillationskolonne 3 wird über Kopf das nicht umgesetzte Ethen C₂ abgezogen, zurück geführt und mit frisch bereitgestellten Einsatzgemisch vermischt.

Vom Sumpf der zweiten Destillationskolonne 3 wird eine C₄-Fraktion C₄ abgezogen enthaltend 1-Buten, cis-2-Buten und trans-2-Buten. Dessen thermische Trennung ist problemlos möglich und erfolgt in einer dritten Destillationskolonne 4. An dem Kopf der dritten Destillationskolonne 4 wird eine dritte Zielfraktion 1B gewonnen, enthaltend das 1-Buten.

Im Sumpf der dritten Destillationskolonne 4 bleiben die beiden 2-Butene, die dort als zweites Intermediat 2B abgezogen werden. Das zweite Intermediat 2B wird einer zweiten Oligomerisierung 5 unterzogen, sodass ein zweites Oligomerisat C₈, C₈₊ anfällt. Dieses enthält begehrte Octene C₈ wie beispielsweise das Di-n-Buten sowie Olefine mit mehr als acht Kohlenstoffatome C₈₊.

Die zweite Oligomerisierung 5 erfolgt in Gegenwart des Festkörpers, der mindestens zwei Komponenten enthält wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus Al₂O₃, SiO₂, TiO₂, ZrO₂ ausgewähltes Metalloxid umfasst.

Die Octene werden als erste Zielfraktion C₈ aus dem zweiten Oligomerisat C₈, C₈₊ gewonnen mit Hilfe einer vierten Destillationskolonne an deren Kopf. In ihrem Sumpf finden sich die Olefine mit mehr als acht Kohlenstoffatome C₈₊ die als Hochsieder aus dem Prozess ausgeschleust werden.

Zurück zu der zweiten Destillationskolonne 3, an deren Sumpf sich die Olefine mit mehr als vier Kohlenstoffatome C₄₊ finden, die in der Ethen-Oligomerisierung 1 entstanden sind. Dazu gehört eine C₆-Fraktion C₆ welche zumindest 1-Hexen, 3-Methyl-cis-2-penten, 3-Methyl-trans-2-penten enthält, darüber hinaus auch 2-Hexen, 3-Hexen und 2-Ethyl-1-buten. Die C₆-Fraktion C₆ wird am Kopf einer fünften Destillationskolonne 7 gewonnen und sodann einer Veretherung 8 unterworfen.

Die Veretherung erfolgt mit Methanol MeOH an einem sauren Ionenaustauschharz. In der Veretherung 8 werden 2-Ethyl-1-buten, 3-Methyl-cis-2-penten, 3-Methyl-trans-2-penten mit dem Methanol selektiv zu 3-Methyl-3-methoxypentan umgesetzt. Dieser Ether wird auch als Methyl-tert.-Hexyl-Ether (MTHxE) bezeichnet. In dem aus der Veretherung 8 abgezogenen ersten Reaktionsgemisch 1H, 2H, 3H, MTHxE findet sich neben dem MTHxE auch nicht umgesetztes 1-Hexen 1H sowie 2-Hexen 2H und 3-Hexen 3H. Diese linearen Hexene 1H, 2H, 3H werden nämlich nicht zu MTHxE verethert.

Die verzweigten C₆-Olefine 2-Ehtyl-1-buten 2EB sowie 3-Methyl-cis-2-penten und 3-Methyl-trans-2-penten 3MP werden hingegen in dem Ether gebunden und können als schwersiedende Etherfraktion MTHxE vom Sumpf einer sechsten Destillationskolonne 9 abgezogen werden.

Erfindungsgemäß erfolgt nun eine Rückspaltung der Etherfraktion MTHxE in ein Gemisch enthaltend 3-Methyl-cis-2-penten, 3-Methyl-trans-2-penten, 2-Ethyl-1-buten sowie Methanol, welches hier als Spaltprodukt MeOH, 2EB, 3MP bezeichnet wird.

Die Spaltreaktion 10 des MTHxE ist die umgekehrte Reaktion der Veretherung 8 und erfolgt in der Gasphase an einem festen Katalysator enthaltend Siliciumdioxid, Aluminiumoxid und Magnesiumoxid. Da die Etherfraktion MTHxE flüssig aus dem Sumpf der sechsten Destillationskolonne 9 abgezogen wird, ist vorher eine Verdampfung erforderlich, die hier aber nicht dargestellt ist.

Das Spaltprodukt MeOH, 2EB, 3MP wird nun in einer siebten Destillationskolonne 11 getrennt in den Alkohol MeOH und in ein erstes Intermediat 2EB, 3MP enthaltend die verzweigten C₆-Oelfine 2-Ethyl-1-buten, 3-Methyl-cis-2-penten und 3-Methyl-trans-2-penten.

Der Alkohol MeOH wird in die Veretherung 8 rezykliert und so vollständig wiederverwertet.

Das erste Intermediat 2EB, 3MP wird gemäß der Lehre der Erfindung in einem separaten Reaktor 12 mit Ethen C₂ umgesetzt. Dabei bilden sich ein zweites Reaktionsgemisch C₈, C₈₊, welches Olefine mit mindestens acht Kohlenstoffatome enthält. Auf diese Weise werden die unerwünschten verzweigten C₆-Olefine 2EB und 3MP in begehrte Octene C₈ umgesetzt.

Die Umsetzung in dem Reaktor 12 erfolgt in Gegenwart desselben Katalysators, der auch in der ersten Oligomerisierung 1 und der zweiten Oligomerisierung 5 eingesetzt wird.

Da sich das zweite Reaktionsgemisch C₈, C₈₊ hinsichtlich seiner Zusammensetzung mit dem aus der zweiten Oligomerisierung 5 erhaltenen zweiten Oligomerisat C₈, C₈₊ und dem Sumpfprodukt C₆₊ der fünften Destillationskolonne 7 ähnelt, werden diese drei Fraktionen gemischt und gemeinsam in der vierten Destillationskolonne 6 aufgearbeitet.

Um die Ausbeute an 1-Hexen zu steigern verfügt die in Figur 1 gezeigte Anlage über eine Isomerisierung 13, welche 2-Hexen und 3-Hexen in 1-Hexen umzusetzen vermag. Dies gelingt an einem heterogenen Katalysator umfassend mindestens eines der Oxide MgO, CaO, BaO, Li₂O, SrO als katalytisch aktives Material.

Die Isomerisierung 13 ist im Sumpfkreislauf einer achten Destillationskolonne 14 angeordnet, an deren Kopf die erste Zielfraktion 1H mit dem 1-Hexen abgezogen wird. Die achte Destillationskolonne 14 wird mit der Kopffraktion der sechsten Destillationskolonne 9 gespeist enthaltend die linearen Hexene 1H, 2H, 3H sowie mit dem aus der Isomerisierung 13 erhaltenen 1-Hexen. Da die Isomerisierung13 in der Gasphase erfolgt, ist im Sumpfkreislauf der achten Destillationskolonne 14 vor der Isomerisierung 13 ein Verdampfer erforderlich, der in der Zeichnung nicht dargestellt ist.

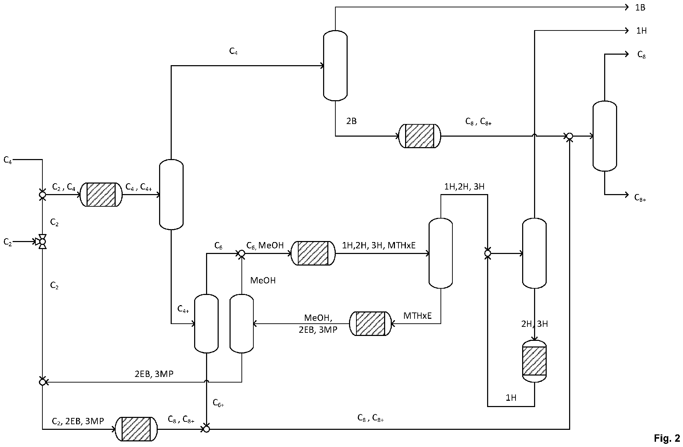

Die in Figur 2 dargestellte Ausführungsform des Verfahrens ist dadurch gekennzeichnet, dass das Einsatzstoffgemisch neben Ethen C₂ auch Olefine mit vier Kohlenstoffatome C₄ enthält, insbesondere 1-Buten, cis-2-Buten und trans-2-Buten. Vor der ersten Oligomerisierung 1 werden diese Rohstoffe so vermischt, dass das Ethen vollständig in dem flüssigen Buten gelöst ist. Die erste Oligomerisierung 1 findet dann in der flüssigen Phase statt. Die Reaktion wird so gesteuert, dass das Ethen vollständig umgesetzt wird, sodass keine separate Abtrennung von nicht umgesetztem Ethen erforderlich ist. Im Falle des Auftretens von Spuren von nicht umgesetztem Ethylen können diese über den Abgasstrom der Destillationskolonne 2 abgeführt werden. Am Kopf der ersten Destillationskolonne 2 wird somit unmittelbar die C₄-Fraktion C₄ gewonnen. Die übrigen Verfahrensschritte sind identisch zu dem von Figur 1.

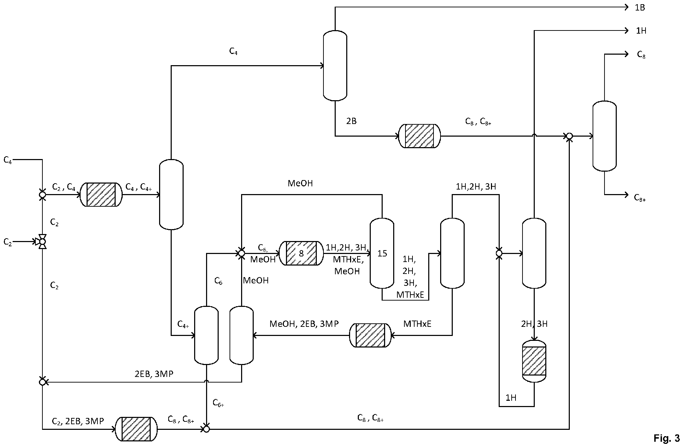

Die in Figur 3 dargestellte Ausführungsform unterscheidet sich von dem in Figur 2 gezeigten Prozess durch eine neunte Destillationskolonne 15. Diese dient dazu, in der Veretherung 8 nicht umgesetztes Methanol MeOH aus dem ersten Reaktionsgemisch abzutrennen und in die Veretherung 8 zurückzuführen. Dies ist insbesondere dann erforderlich, wenn Methanol überstöchiometrisch in die Veretherung gefahren wird.

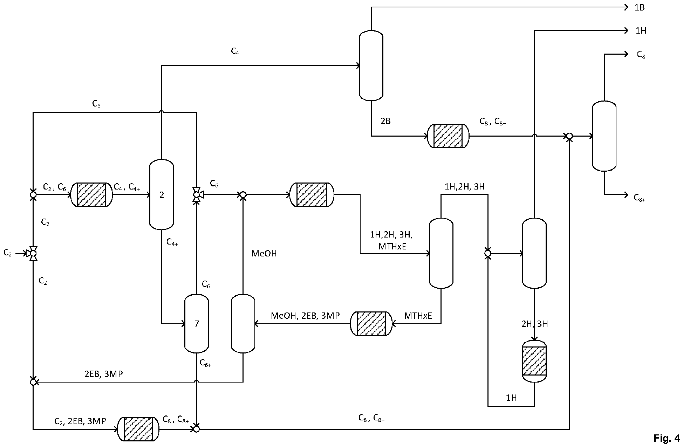

Figur 4 zeigt eine Ausführungsform, bei der ein Teil am Kopf der fünften Destillationskolonne 7 gewonnenen C₆-Fraktion C₆ abgezweigt und gemeinsam mit dem Ethen C₂ der ersten Oligomerisierung 1 zugeführt wird. Der andere Teil der C6-Fraktion wird der Veretherung 8 zugeführt. Das zurückgeführte C₆-Oelfingemisch C₆ dient als Lösemittel für das Ethen C₂, sodass die erste Oligomerisierung 1 in der Flüssigphase durchgeführt werden kann. Das Ethen wird in dem flüssigen C6-Olefin gelöst. Da das C6-Olefin in der Oligomerisierung reaktiv ist, wird entsprechend mehr C8 gebildet. Die Ausbeute an Hexen 1H fällt im Ergebnis niedriger aus. Der Vorteil dieser Verfahrensvariante ist, dass es einzig mit dem Rohstoff Ethen auskommt, denn das reaktive Lösemittel C₆ wird in dem Prozess intern gebildet. Bei dem in Figur 3 gezeigten Prozess muss das reaktive Lösemittel C₄ als Rohstoff hingegen von außen zugeführt werden.

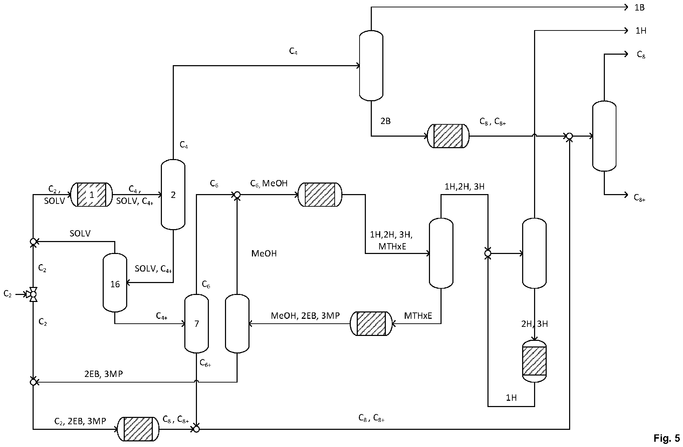

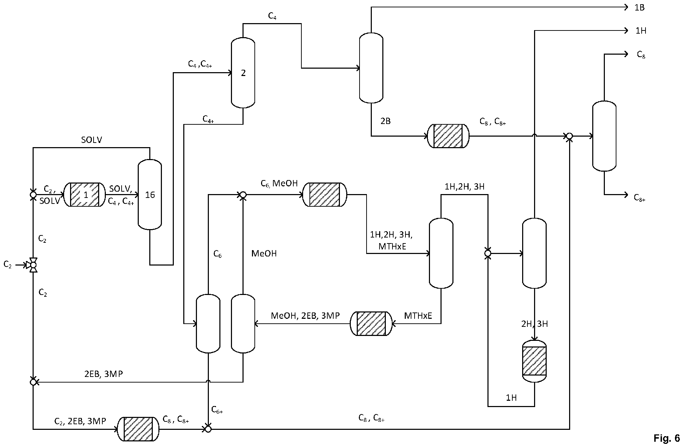

Die Figuren 5 und 6 zeigen jeweils eine Verfahrensvariante, die mit einem inerten Lösemittel SOLV arbeitet, welches im Kreis geführt wird und deswegen ebenfalls nicht von außen zugeführt werden muss.

In Figur 5 wird ein verglichen mit Butenen schwer siedendes, inertes Lösemittel SOLV wie n-Heptan oder n-Hexan verwendet. Das Ethen C₂ wird darin vollständig gelöst, sodass die erste Oligomerisierung 1 in der Flüssigphase erfolgt. Da sich das Lösemittel SOLV in der Oligomerisierung inert verhält, wird es nicht umgesetzt und findet sich im ersten Oligomerisat C₄, SOLV, C₄₊ wieder. In der ersten Destillationskolonne 2 gerät es in den Sumpf, da es einen höheren Siedepunkt hat als die C₄-Olefine C₄, die in der ersten Kolonne 2 über Kopf gehen. Um das Lösemittel SOLV von den Olefinen mit mehr als vier Kohlenstoffatome C₄₊ zu trennen, ist eine zehnte Destillationskolonne 16 vorgesehen. Diese trennt das Lösemittel SOLV über Kopf ab, sodass es vollständig wiedergewonnen und frisches Ethen C₂ darin gelöst werden kann. Vom Sumpf der zehnten Destillationskolonne 16 werden die Olefine mit mehr als vier Kohlenstoffatome C₄₊ abgezogen und wie bei den anderen Ausführungsformen derfünften Destillationskolonne 7 zugeführt.

In Figur 6 wird ein verglichen mit Butenen leicht siedendes, inertes Lösemittel SOLV wie Propan oder Isobutan verwendet. Das Ethen C₂ wird darin vollständig gelöst, sodass die erste Oligomerisierung 1 in der Flüssigphase erfolgt. Da sich das Lösemittel SOLV in der Oligomerisierung inert verhält, wird es nicht umgesetzt und findet sich im ersten Oligomerisat SOLV, C₄, C₄₊ wieder. Da es leichter siedet als die die C₄-Olefine C₄ ist die zehnte Destillationskolonne 16 direkt hinter der ersten Oligomerisierung 1 angeordnet. Darin wird das Lösemittel SOLV über Kopf abgetrennt und rezykliert. Der Sumpf der zehnten Destillationskolonne 16 wird der ersten Destillationskolonne 2 und dort so aufgearbeitet wie bei den anderen Ausführungsformen.

Letztendlich ist die Anordnung der ersten Destillationskolonne 1 und der zehnten Destillationskolonne 16 bei den Ausführungsformen in den Figuren 5 und 6 vertauscht, da das Lösemittel einmal bei höherer Temperatur (Figur 5) und einmal bei niedrigerer Temperatur (Figur 6) als die Butene siedet.

### Bezugszeichenliste

- 1: erste Oligomerisierung
- 2: erste Destillationskolonne
- 3: zweite Destillationskolonne
- 4: dritte Destillationskolonne
- 5: zweite Oligomerisierung
- 6: vierte Destillationskolonne
- 7: fünfte Destillationskolonne
- 8: Veretherung
- 9: sechste Destillationskolonne
- 10: Spaltreaktion
- 11: siebte Destillationskolonne
- 12: Reaktor
- 13: Isomerisierung
- 14: achte Destillationskolonne
- 15: neunte Destillationskolonne
- 16: zehnte Destillationskolonne
- C₂: Ethen (Einsatzstoffgemisch)
- C₂₊: Olefine mit mehr als zwei Kohlenstoffatomen
- C₄: C₄-Fraktion enthaltend Olefine mit vier Kohlenstoffatomen
- C₄₊: Olefine mit mehr als vier Kohlenstoffatomen
- C₂, C₂₊: erstes Oligomerisat
- C₆:: C₆-Fraktion enthaltend Olefine mit sechs Kohlenstoffatomen
- C₆₊: C₆₊-Fraktion enthaltend Olefine mit mehr als sechs Kohlenstoffatomen
- C₈:: Octen (zweite Zielfraktion)
- C₈₊: C₆₊-Fraktion enthaltend Olefine mit mehr als sechs Kohlenstoffatomen
- C₈, C₈+: zweites Oligomerisat, zweites Reaktionsgemisch
- 1B: 1-Buten (dritte Zielfraktion)
- 2B: Zweites Intermediat enthaltend cis-2-Buten und trans-2-Buten
- 1H: 1-Hexen (erste Zielfraktion)
- 2H: 2-Hexen
- 3H: 3-Hexen
- MeOH:: Methanol
- MTHxE:: Etherfraktion, enthaltend 3-Methyl-3-methoxypentan;
- 1H, 2H, 3H, MTHxE: erstes Reaktionsgemisch
- 2EB: 2-Ethyl-1-buten
- 3MP: 3-Methyl-cis-2-penten und 3-Methyl-trans-2-penten
- MeOH, 2EB, 3MP: Spaltprodukt
- 2EB, 3MP: Erstes Intermediat
- SOLV: Lösemittel

## Patentansprüche

1. Verfahren zur Herstellung von zumindest 1-Hexen und Octen aus Ethen mit den folgenden Schritten:
a) Bereitstellen eines Einsatzstoffgemisches enthaltend Ethen;
b) Unterwerfen des Ethens einer ersten Oligomerisierung, wobei ein erstes Oligomerisat erhalten wird, welches Olefine mit weniger als sechs Kohlenstoffatome, zumindest die C₆-Olefine 1-Hexen, 3-Methyl-cis-2-penten, 3-Methyl-trans-2-penten und Olefine mit mehr als sechs Kohlenstoffatome enthält,
c) Destillatives Aufarbeiten des ersten Oligomerisats, wobei eine C₆-Fraktion gewonnen wird, die zumindest 1-Hexen, 3-Methyl-cis-2-penten, 3-Methyl-trans-2-penten enthält,
d) Unterwerfen der C₆-Fraktion einer Veretherung unter Zugabe eines Alkohols in Gegenwart eines heterogenen Katalysators unter Erhalt eines ersten Reaktionsgemisches, enthaltend zumindest 1-Hexen und 3-Methyl-3-methoxypentan;
e) Destillatives Aufarbeiten des ersten Reaktionsgemisches unter Erhalt von einer ersten Zielfraktion enthaltend 1-Hexen sowie einer Etherfraktion mindestens enthaltend 3-Methyl-3-methoxypentan;
wobei die Verbesserung darin besteht:
f) die Etherfraktion wird einer Spaltreaktion unterworfen, wobei ein Spaltprodukt erhalten wird, welches zumindest 3-Methyl-cis-2-penten, 3-Methyl-trans-2-penten sowie den Alkohol enthält;
g) der Alkohol wird aus dem Spaltprodukt destillativ abgetrennt und in der Veretherung wiederverwertet;
h) ein erstes Intermediat enthaltend zumindest 3-Methyl-cis-2-penten, 3-Methyl-trans-2-penten wird aus dem Spaltprodukt destillativ abgetrennt;
i) das erste Intermediat wird mit Ethen umgesetzt, sodass ein zweites Reaktionsgemisch erhalten wird, welches Olefine mit zumindest acht Kohlenstoffatome enthält;
k) eine zweite Zielfraktion enthaltend Octen wird aus dem zweiten Reaktionsgemisch destillativ gewonnen,
wobei mindestens zwei Komponenten enthaltender Festkörper in der ersten Oligomerisierung und/oder in der zweiten Oligomerisierung als heterogener Katalysator eingesetzt wird, wobei die erste Komponente mindestens ein aus Ni, Fe, Ti ausgewähltes Element umfasst, welches in metallischer und/oder oxidischer und/oder hydridischer Form vorliegt, und wobei die zweite Komponente mindestens ein aus Al₂O₃, SiO₂, TiO₂, ZrO₂ ausgewähltes Metalloxid umfasst.

2. Verfahren nach Anspruch 1, bei welchem neben 1-Hexen und Octen auch 1-Buten folgendermaßen hergestellt wird:
a) die erste Oligomerisierung wird dergestalt durchgeführt, dass das erste Oligomerisat 1-Buten, cis-2-Buten und trans-2-Buten enthält;
b) beim destillativen Aufarbeiten des ersten Oligomerisats wird eine C₄-Fraktion gewonnen, die 1-Buten, cis-2-Buten und trans-2-Buten enthält;
c) die C₄-Fraktion wird destillativ getrennt in eine dritte Zielfraktion enthaltend 1-Buten und in ein zweites Intermediat, welches cis-2-Buten und trans-2-Buten enthält;
d) das zweite Intermediat wird einer zweiten Oligomerisierung unterworfen, wobei ein zweites Oligomerisat erhalten wird, welches Olefine mit zumindest acht Kohlenstoffatome enthält;
e) das zweite Oligomerisat wird gemeinsam mit dem zweiten Reaktionsgemisch destillativ aufgearbeitet, wobei die zweite Zielfraktion gewonnen wird.

3. Verfahren nach Anspruch 1, **dadurch gekennzeichnet, dass** beim destillativen Aufarbeiten des ersten Oligomerisats eine C₆₊-Fraktion gewonnen wird, die Olefine mit mehr als sechs Kohlenstoffatome enthält, und dass die C₆₊-Fraktion gemeinsam dem zweiten Reaktionsgemisch destillativ aufgearbeitet wird, wobei die zweite Zielfraktion gewonnen wird.

4. Verfahren nach Anspruch 2, **dadurch gekennzeichnet, dass** beim destillativen Aufarbeiten des ersten Oligomerisats eine C₆₊-Fraktion gewonnen wird, die Olefine mit mehr als sechs Kohlenstoffatome enthält, und dass die C₆₊-Fraktion gemeinsam dem zweiten Reaktionsgemisch und mit dem zweiten Oligomerisat destillativ aufgearbeitet wird, wobei die zweite Zielfraktion gewonnen wird.

5. Verfahren nach einem der Ansprüche 1 bis 4, **dadurch gekennzeichnet, dass** das Einsatzstoffgemisch mehr als 95 Gew.-% Ethen enthält, und dass die Reaktionsbedingungen der ersten Oligomerisierung so gewählt sind, dass die erste Oligomerisierung in der Gasphase in Gegenwart eines heterogenen Katalysators erfolgt.

6. Verfahren nach einem der Ansprüche 1 bis 4, **dadurch gekennzeichnet, dass** das Einsatzstoffgemisch weniger als 25 Gew.-% Ethen enthält, dass das Einsatzstoffgemisch mehr als 75 Gew.-% eines Lösemittels enthält, wobei die Reaktionsbedingungen der ersten Oligomerisierung und die Konzentration des Ethens in dem Lösemittel so gewählt sind, dass die erste Oligomerisierung in der flüssigen Phase erfolgt und das Ethen in dem flüssigen Lösemittel vollständig gelöst ist.

7. Verfahren nach Anspruch 5 oder 6, **dadurch gekennzeichnet, dass** in der ersten Oligomerisierung nicht umgesetztes Ethen destillativ aus dem ersten Oligomerisat abgetrennt und in die erste Oligomerisierung zurückgeführt wird.

8. Verfahren nach einem der Ansprüche 1 bis 4, **dadurch gekennzeichnet, dass** das Einsatzstoffgemisch neben Ethen auch Olefine mit vier Kohlenstoffatome enthält, wobei die Reaktionsbedingungen der ersten Oligomerisierung und die Zusammensetzung des Einsatzstoffgemisches so gewählt sind, dass die erste Oligomerisierung in der flüssigen Phase erfolgt und das Ethen in den Olefinen mit vier Kohlenstoffatomen vollständig gelöst ist.

9. Verfahren nach einem der Ansprüche 1 bis 4, **dadurch gekennzeichnet, dass** das Einsatzstoffgemisch neben Ethen auch Olefine mit sechs Kohlenstoffatome enthält, wobei die Reaktionsbedingungen der ersten Oligomerisierung und die Zusammensetzung des Einsatzstoffgemisches so gewählt sind, dass die erste Oligomerisierung in der flüssigen Phase erfolgt und das Ethen in den Olefinen mit sechs Kohlenstoffatomen vollständig gelöst ist.

10. Verfahren nach einem der Ansprüche 1 bis 4, **dadurch gekennzeichnet, dass** das Einsatzstoffgemisch neben Ethen auch Olefine mit vier Kohlenstoffatome und Olefine mit sechs Kohlenstoffatome enthält, wobei die Reaktionsbedingungen der ersten Oligomerisierung und die Zusammensetzung des Einsatzstoffgemisches so gewählt sind, dass die erste Oligomerisierung in der flüssigen Phase erfolgt und das Ethen in den Olefinen mit vier und sechs Kohlenstoffatomen vollständig gelöst ist.

11. Verfahren nach einem der Ansprüche 1 bis 10, **dadurch gekennzeichnet, dass** die Spaltreaktion in der Gasphase bei einem Druck von 1*10⁵ Pa bis 25*10⁵ Pa und einer Temperatur von 130°C bis 350°C Gegenwart von einem Festkörper erfolgt, welcher die folgende, sich zu 100 Gew.-% ergänzende Zusammensetzung aufweist:
| | |
|---|---|
| • Siliciumdioxid: | 61 Gew.-% bis 74 Gew.-%; |
| • Aluminiumdioxid: | 19 Gew.-% bis 23 Gew.-%; |
| • Magnesiumoxid: | 10 Gew.-% bis 12 Gew.-%; |
| • Summe sonstige Stoffe: | 0 bis 10 Gew.-%. |

12. Verfahren nach einem der Ansprüche 1 bis 11, **dadurch gekennzeichnet, dass** die Veretherung in der flüssigen Phase bei einem Druck von 1*10⁵ Pa bis 7*10⁵ Pa und einer Temperatur von 50°C bis 130°C in Gegenwart eines Polystyrol-Divinylbenzol-Copolymers erfolgt, welches Sulfonsäuregruppen oder Carbonsäuregruppen trägt.

## Claims

1. Process for producing at least 1-hexene and octene from ethene comprising the steps of:
a) providing a feedstock mixture comprising ethene;
b) subjecting the ethene to a first oligomerization to obtain a first oligomerizate which comprises olefins having less than six carbon atoms, at least the C₆ olefins 1-hexene, 3-methyl-cis-2-pentene, 3-methyl-trans-2-pentene and olefins having more than six carbon atoms,
c) distillative workup of the first oligomerizate to obtain a C₆ fraction which comprises at least 1-hexene, 3-methyl-cis-2-pentene, 3-methyl-trans-2-pentene,
d) subjecting the C₆ fraction to an etherification by addition of an alcohol in the presence of a heterogeneous catalyst to obtain a first reaction mixture comprising at least 1-hexene and 3-methyl-3-methoxypentane;
e) distillative workup of the first reaction mixture to obtain a first target fraction comprising 1-hexene and an ether fraction at least comprising 3-methyl-3-methoxypentane;
wherein the improvement consists in:
f) the ether fraction is subjected to a cleavage reaction to obtain a cleavage product which comprises at least 3-methyl-cis-2-pentene, 3-methyl-trans-2-pentene and the alcohol;
g) the alcohol is removed from the cleavage product by distillation and reused in the etherification;
h) a first intermediate comprising at least 3-methyl-cis-2-pentene, 3-methyl-trans-2-pentene is removed from the cleavage product by distillation;
i) the first intermediate is reacted with ethene to obtain a second reaction mixture which comprises olefins having at least eight carbon atoms;
k) a second target fraction comprising octene is obtained from the second reaction mixture by distillation,
wherein a solid body comprising at least two components is employed as a heterogeneous catalyst in the first oligomerization and/or in the second oligomerization, wherein the first component comprises at least one element selected from Ni, Fe, Ti which is present in metallic and/or oxidic and/or hydridic form and wherein the second component comprises at least one metal oxide selected from Al₂O₃, SiO₂, TiO₂, ZrO₂.

2. Process according to Claim 1, wherein not only 1-hexene and octene but also 1-butene are produced as follows:
a) the first oligomerization is performed such that the first oligomerizate comprises 1-butene, cis-2-butene and trans-2-butene;
b) distillative workup of the first oligomerizate affords a C₄ fraction which comprises 1-butene, cis-2-butene and trans-2-butene;
c) the C₄ fraction is separated by distillation into a third target fraction comprising 1-butene and into a second intermediate which comprises cis-2-butene and trans-2-butene;
d) the second intermediate is subjected to a second oligomerization to obtain a second oligomerizate which comprises olefins having at least eight carbon atoms;
e) the second oligomerizate is worked up by distillation together with the second reaction mixture to obtain the second target fraction.

3. Process according to Claim 1, **characterized in that** the distillative workup of the first oligomerizate affords a C₆₊ fraction which comprises olefins having more than six carbon atoms and **in that** the C₆₊ fraction is worked up by distillation together with the second reaction mixture to obtain the second target fraction.

4. Process according to Claim 2, **characterized in that** the distillative workup of the first oligomerizate affords a C₆₊ fraction which comprises olefins having more than six carbon atoms and **in that** the C₆₊ fraction is worked up by distillation together with the second reaction mixture and with the second oligomerizate to obtain the second target fraction.

5. Process according to any of Claims 1 to 4, **characterized in that** the feedstock mixture comprises more than 95 wt% of ethene and that the reaction conditions of the first oligomerization are chosen such that the first oligomerization is effected in the gas phase in the presence of a heterogeneous catalyst.

6. Process according to any of Claims 1 to 4, **characterized in that** the feedstock mixture comprises less than 25 wt% of ethene, **in that** the feedstock mixture comprises more than 75 wt% of a solvent, wherein the reaction conditions of the first oligomerization and the concentration of the ethene in the solvent are chosen such that the first oligomerization is effected in the liquid phase and the ethene is fully dissolved in the liquid solvent.

7. Process according to Claim 5 or 6, **characterized in that** ethene unconverted in the first oligomerization is removed by distillation from the first oligomerizate and recycled into the first oligomerization.

8. Process according to any of Claims 1 to 4, **characterized in that** the feedstock mixture comprises not only ethene but also olefins having four carbon atoms, wherein the reaction conditions of the first oligomerization and the composition of the feedstock mixture are chosen such that the first oligomerization is effected in the liquid phase and the ethene is fully dissolved in the olefins having four carbon atoms.

9. Process according to any of Claims 1 to 4, **characterized in that** the feedstock mixture comprises not only ethene but also olefins having six carbon atoms, wherein the reaction conditions of the first oligomerization and the composition of the feedstock mixture are chosen such that the first oligomerization is effected in the liquid phase and the ethene is fully dissolved in the olefins having six carbon atoms.

10. Process according to any of Claims 1 to 4, **characterized in that** the feedstock mixture comprises not only ethene but also olefins having four carbon atoms and olefins having six carbon atoms, wherein the reaction conditions of the first oligomerization and the composition of the feedstock mixture are chosen such that the first oligomerization is effected in the liquid phase and the ethene is fully dissolved in the olefins having four and six carbon atoms.

11. Process according to any of Claims 1 to 10, **characterized in that** the cleavage reaction is effected in the gas phase at a pressure of 1*10⁵ Pa to 25*10⁵ Pa and a temperature of 130°C to 350°C in the presence of a solid body having the following composition which sums to 100 wt%:
• silicon dioxide: 61 wt% to 74 wt%;
• aluminum oxide: 19 wt% to 23 wt%;
• magnesium oxide: 10 wt% to 12 wt%;
• sum of other substances: 0 to 10 wt%.

12. Process according to any of Claims 1 to 11, **characterized in that** the etherification is effected in the liquid phase at a pressure of 1*10⁵ Pa to 7*10⁵ Pa and a temperature of 50°C to 130°C in the presence of a polystyrene-divinylbenzene copolymer bearing sulfonic acid groups or carboxylic acid groups.

## Revendications

1. Procédé pour la préparation d'au moins 1-hexène et octène à partir d'éthylène selon les étapes suivantes :
a) mise à disposition d'un mélange de matières à traiter contenant de l'éthylène ;
b) soumission de l'éthylène à une première oligomérisation, un premier oligomère étant obtenu, qui contient des oléfines comportant moins de six atomes de carbone, au moins les oléfines en C₆ 1-hexène, 3-méthyl-cis-2-pentène, 3-méthyl-trans-2-pentène et des oléfines comportant plus de six atomes de carbone,
c) le traitement par distillation du premier oligomère, une fraction C₆ étant obtenue, qui contient au moins du 1-hexène, du 3-méthyl-cis-2-pentène, du 3-méthyl-trans-2-pentène,
d) la soumission de la fraction C₆ à une éthérification par ajout d'un alcool en présence d'un catalyseur hétérogène avec obtention d'un premier mélange réactionnel, contenant au moins du 1-hexène et du 3-méthyl-3-méthoxypentane ;
e) le traitement par distillation du premier mélange réactionnel avec obtention d'une première fraction cible contenant du 1-hexène ainsi qu'une fraction éthérée contenant au moins du 3-méthyl-3-méthoxypentane ;
l'amélioration résidant dans le fait que :
f) la fraction éthérée est soumise à une réaction de fission, un produit de fission étant obtenu, qui contient au moins du 3-méthyl-cis-2-pentène, du 3-méthyl-trans-2-pentène ainsi qu'un alcool ;
g) l'alcool est séparé du produit de fission par distillation et est valorisé à nouveau dans l'éthérification ;
h) un premier intermédiaire contenant au moins du 3-méthyl-cis-2-pentène, du 3-méthyl-trans-2-pentène est séparé du produit de fission par distillation ;
i) le premier intermédiaire est transformé avec de l'éthylène, de sorte qu'un deuxième mélange réactionnel est obtenu, qui contient des oléfines comportant au moins 8 atomes de carbone ;
k) une deuxième fraction cible contenant de l'octène est obtenue par distillation à partir du deuxième mélange réactionnel,
au moins deux solides contenant des composants étant utilisés en tant que catalyseur hétérogène dans la première oligomérisation et/ou dans la deuxième oligomérisation, le premier composant comprenant au moins un élément choisi parmi Ni, Fe, Ti, qui se trouve sous forme métallique et/ou d'oxyde et/ou d'hydrure, et le deuxième composant comprenant au moins un oxyde métallique choisi parmi Al₂O₃, SiO₂, TiO₂, ZrO₂.

2. Procédé selon la revendication 1, dans lequel, mis à part du 1-hexène et de l'octène, également du 1-butène est également préparé comme suit :
a) la première oligomérisation est réalisée de telle sorte que le premier oligomère contient du 1-butène, du cis-2-butène et du trans-2-butène ;
b) lors du traitement par distillation du premier oligomère, une fraction C₄ est obtenue, qui contient du 1-butène, du cis-2-butène et du trans-2-butène ;
c) la fraction C₄ est séparée par distillation en une troisième fraction cible contenant du 1-butène et en un deuxième intermédiaire, qui contient du cis-2-butène et du trans-2-butène ;
d) le deuxième intermédiaire est soumis à une deuxième oligomérisation, un deuxième oligomère étant obtenu, qui contient des oléfines comportant au moins huit atomes de carbone ;
e) le deuxième oligomère est traité par distillation conjointement avec le deuxième mélange réactionnel, la deuxième fraction cible étant obtenue.

3. Procédé selon la revendication 1, **caractérisé en ce que**, lors du traitement par distillation du premier oligomère, une fraction C₆₊ est obtenue, qui contient des oléfines comportant plus de six atomes de carbone, et **en ce que** la fraction C₆₊ est traitée par distillation conjointement avec le deuxième mélange réactionnel, la deuxième fraction cible étant obtenue.

4. Procédé selon la revendication 2, **caractérisé en ce que**, lors du traitement par distillation du premier oligomère, une fraction C₆₊ est obtenue, qui contient des oléfines comportant plus de six atomes de carbone, et **en ce que** la fraction C₆₊ est traitée par distillation conjointement avec le deuxième mélange réactionnel et avec le deuxième oligomère, la deuxième fraction cible étant obtenue.

5. Procédé selon l'une quelconque des revendications 1 à 4, **caractérisé en ce que** le mélange de matières à traiter contient plus de 95 % en poids d'éthylène, et **en ce que** les conditions de réaction de la première oligomérisation sont choisies de sorte que la première oligomérisation a lieu en phase gazeuse en présence d'un catalyseur hétérogène.

6. Procédé selon l'une quelconque des revendications 1 à 4, **caractérisé en ce que** le mélange de matières à traiter contient moins de 25 % en poids d'éthylène, **en ce que** le mélange de matières à traiter contient plus de 75 % en poids d'un solvant, les conditions de réaction de la première oligomérisation et la concentration de l'éthylène dans le solvant étant choisies de sorte que la première oligomérisation a lieu en phase liquide et l'éthylène est totalement dissous dans le solvant liquide.

7. Procédé selon la revendication 5 ou 6, **caractérisé en ce que** dans la première oligomérisation, l'éthylène n'ayant pas été transformé est séparé du premier oligomère par distillation et reconduit dans la première oligomérisation.

8. Procédé selon l'une quelconque des revendications 1 à 4, **caractérisé en ce que** le mélange de matières à traiter, contient, mis à part l'éthylène, aussi des oléfines comportant quatre atomes de carbone, les conditions de réaction de la première oligomérisation et la composition du mélange de matières à traiter étant choisies de sorte que la première oligomérisation a lieu en phase liquide et l'éthylène est totalement dissous dans les oléfines comportant quatre atomes de carbone.

9. Procédé selon l'une quelconque des revendications 1 à 4, **caractérisé en ce que** le mélange de matières à traiter, contient, mis à part l'éthylène, aussi des oléfines comportant six atomes de carbone, les conditions de réaction de la première oligomérisation et la composition du mélange de matières à traiter étant choisies de sorte que la première oligomérisation a lieu en phase liquide et l'éthylène est totalement dissous dans les oléfines comportant six atomes de carbone.

10. Procédé selon l'une quelconque des revendications 1 à 4, **caractérisé en ce que** le mélange de matières à traiter, contient, mis à part l'éthylène, aussi des oléfines comportant quatre atomes de carbone et des oléfines comportant six atomes de carbone, les conditions de réaction de la première oligomérisation et la composition du mélange de matières à traiter étant choisies de sorte que la première oligomérisation a lieu en phase liquide et l'éthylène est totalement dissous dans les oléfines comportant quatre et six atomes de carbone.

11. Procédé selon l'une quelconque des revendications 1 à 10, **caractérisé en ce que** la réaction de fission a lieu en phase gazeuse à une pression de 1*10⁵ Pa à 25*10⁵ Pa et à une température de 130 °C à 350 °C en présence d'un solide, qui présence la composition suivante qui s'étend jusqu'à 100 % en poids :
• dioxyde de silicium : 61 % en poids à 74 % en poids ;
• dioxyde d'aluminium : 19 % en poids à 23 % en poids ;
• oxyde de magnésium : 10 % en poids à 12 % en poids ;
• somme des autres matières : 0 à 10 % en poids.

12. Procédé selon l'une quelconque des revendications 1 à 11, **caractérisé en ce que** l'éthérification a lieu en phase liquide à une pression de 1*10⁵ Pa à 7*10⁵ Pa et à une température de 50 °C à 130 °C en présence d'un copolymère polystyrène-divinylbenzène, qui porte des groupes acide sulfonique ou des groupes acide carboxylique.
